# EUROPEAN PATENT APPLICATION

(11) **EP 2 236 078 A1**
(43) Date of publication of application: **06.10.2010**
(21) Application number: 09157183.6
(22) Date of filing: 02.04.2009
(51) Int. Cl.: A61B 5/0476, A61B 5/0488, A61B 5/0496

(54) **Processing a bio-physiological signal**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to an apparatus and a method for processing at least one bio-physiological signal of a sleeping object, such as a brain wave signal or electroencephalography (EEG) signal, an electro-oculogram (EOG) signal, or an electromyogram (EMG) signal. The signal is captured by at least one sensor, which may be included in a pliable device such as a pillow or headgear. A processing unit (18) processes the at least one bio-physiological signal and thereby generates an output signal (28; 38; 44) based on the at least one bio-physiological signal and based on a signal pattern stored or generated in the processing unit (18), the output signal (28; 38; 44) comprising a temporally varying output signal pattern, wherein the output signal pattern depends on a current sleep state of the sleeping object. The output signal may be reproduced in a human-perceptible form.

## Description

### FIELD OF THE INVENTION

The invention relates to the field of processing bio-physiological signals, and more specifically to an apparatus for processing a bio-physiological signal and to a method of processing a bio-physiological signal. In particular, the bio-physiological signal may be a brain wave signal.

### BACKGROUND OF THE INVENTION

In the sleep of a human being, different sleep stages are distinguished by typical bio-physiological signals or signal patterns associated with the respective sleep stages.

In the context of this application, the term "sleep state" or "sleep stage" is considered to exclude an awake state.

Whereas in the brain of an awake and relaxed person, oscillations at about 10 Hz (alpha waves) are present in electroencephalography signals, other patterns of brain wave signals are observed in respective sleep stages. An alert person generates beta waves which are about twice as fast as alpha waves.

In stage 1 sleep, being a transition stage between wake and sleep, theta waves having a frequency typically in the range of 3.5 to 7 Hz are present.

In stage 2 sleep, so called sleep spindles having a frequency of 12 to 16 Hz and "K-complexes" are observed.

In slow wave sleep, also known as stage 3 and stage 4 sleep, delta waves, typically in the range of 0.5 to 4 Hz, are present. Moreover, the amplitude of the brain wave signal is increased as compared to other sleep stages. This sleep stage is also called delta sleep and is the "deepest stage of sleep".

The sleep stages 1, 2, and slow wave sleep may be summarized as non-rapid eye movement (NREM) or "non-REM" sleep, and are distinguished from rapid eye movement (REM) sleep. During the REM sleep stage, rapid low amplitude brain wave signals are observed. This sleep stage is generally associated with the sleeping person experiencing dreams.

The REM sleep stage may also be distinguished from other sleep stages from the associated electro-oculogram signals and electro-myogram signals. During REM sleep, the sleeping person looses his ability to use postural or skeletal muscles, and this muscle inactivity can be determined from electro-myogram signals. The REM sleep stage is also characterised by rapid movements of the eyes, which clearly distinguishes REM sleep from NON-REM sleep in the electro-oculogram signals.

From WO2005/084538 A1, a device and a method for waking a user in a desired sleep state are known. The user's sleep state may be monitored during the night or sleep experience. Electrodes may be placed against the skin of the user to monitor an electroencephalogram (EEG) signal, an electro-oculogram (EOG) signal and/or an electro-myogram (EMG) signal. The sleep state of the user is determined using a sleep state detection algorithm to process information from the electrodes. The device may predict an occurrence when the user will be in the desired sleep state, such as light sleep, and wake the user during that predicted occurrence.

### SUMMARY OF THE INVENTION

It would be desirable for many people to be able to recognize a sleep state of another person. For example, it would be desirable to provide an apparatus or a method that is able to indicate whether a sleeping object is deep asleep or whether a person is dreaming.

To better address one or more of these concerns, in a first aspect of the invention, an apparatus for processing a bio-physiological signal is provided that comprises:
- at least one sensor for capturing at least one bio-physiological signal of a sleeping object; and
- a processing unit for processing the at least one bio-physiological signal and thereby generating an output signal based on the at least one bio-physiological signal and based on a signal pattern stored or generated in the processing unit, the output signal comprising a temporally varying output signal pattern, wherein the output signal pattern depends on a current sleep state of the sleeping object. For example, for at least two different sleep states of the sleeping object, different temporally varying output signal patterns of the output signal are generated.

In the context of the present application, the term "sleeping object" covers human beings and animals, in particular endotherms, and, more specifically, mammals. In particular, the sleeping object may be a sleeping person.

The bio-physiological signal is, for example, a brain wave signal or electroencephalography (EEG) signal, an electro-oculogram (EOG) signal, or an electro-myogram (EMG) signal. For example, muscle tone may be measured as an electro-myogram signal, and eye movement may be measured as an electro-oculogram signal. Preferably, the bio-physiological signal is a brain wave signal.

The term "brain wave signal" is to be understood to mean an electromagnetic signal produced by or correlated to electrical activity of the brain, i.e., the firing of neurons within the brain. For example, the brain wave signal may be an electroencephalography signal.

Further, the term "capturing a bio-physiological signal" covers measuring an electrical signal using electrodes on the skin of the sleeping object as well as contactlessly detecting an electromagnetic signal in the vicinity of the sleeping object. Further, the term "capturing a brain wave signal" covers measuring an electrical signal using electrodes on the scalp of the sleeping object as well as contactlessly detecting an electromagnetic signal in the vicinity of the head of the sleeping object. Since the bio-physio logical signals are not captured for clinical applications, contactless measurements of the bio-physiological signals are possible.

The term "a temporally varying output signal pattern" is to be understood as an output signal or an output signal component having a temporally varying characteristic, for example a pattern of varying output signal intensity, a pattern of varying color and/or intensity at least one position of a two-dimensional graphical signal, or a two-dimensional graphical pattern that varies in time. However, the term is not limited to these examples.

Because the temporally varying output signal pattern depends on a current sleep state of the sleeping object, for example, different sleep states may be recognized or distinguished by an observer to which the output signal is reproduced in a human-perceptible form. Furthermore, for example, an observer may be entertained by or may enjoy perceiving the output signal. For example, for at least two different sleep states of the sleeping object, different temporally varying output signal patterns of the output signal are generated. For example, the output signal may reflect whether the sleeping object is dreaming or whether the sleeping object is deeply asleep.

The apparatus preferably is a consumer product, in particular an entertainment device. For example, the apparatus is a portable apparatus.

For example, the processing unit is adapted to permanently output the output signal and/or to permanently output the output signal at least during one sleep state of the sleeping object. For example, the processing unit is adapted to permanently output the output signal at least during two different sleep states of the sleeping object, wherein a respective output signal pattern depends on the current sleep state of the sleeping object.

For example, the apparatus comprises an output unit for reproducing the output signal in human-perceptible form. The term "human-perceptible form" covers a signal that can be directly perceived by a person, e.g. an audible signal and/or a visible signal. For example, the output unit may visualize a bio-physiological signal in a form that provides meaning to a person that observes it. This may be done in an aesthetic manner.

For example, the output signal is synchronized to or correlated to at least one bio-physiological signal captured by at least one sensor. For example, the output signal may be synchronized or correlated with a brain wave signal in so far as a currently present main frequency of a brain wave signal, such as the frequency of theta waves or delta waves, may be present or detectable in the output signal.

The term "correlated" is to be understood as requiring that momentary slight variations of wave frequency of a currently present main frequency of the bio-physiological signal are reproduced as slight variations of wave frequency of that same frequency being present or detectable in the output signal. In particular, these variations may be concurrently reproduced in the output signal, wherein a time lag e.g. caused by digitalization of signals or digital processing may nevertheless be present.

For example, an intensity pattern of a brain wave signal, such as a wave pattern during slow wave sleep, may have a base frequency within a range of less than 3.5 Hz, and said frequency may also appear in the output signal. Thus, for example, a direct visualization of brain activity may be provided combined with aesthetic features of the output signal.

For example, the apparatus further comprises a pliable device that comprises said at least one sensor. Such a pliable device may be positioned at, around or below the head of the sleeping object, for example, without causing discomfort to the sleeping object. In particular, for example, the apparatus may comprise a single pliable device for each sleeping object, said single pliable device comprising one or more sensors for capturing a least one brain wave signal of the sleeping object. In contrast to the measuring of EEG signals for clinical applications, requirements on the quality of the captured brain wave signal(s) may be less strict for an apparatus of the invention. Therefore, wet electrodes placed on the scalp of the sleeping object can be dispensed with, and a more flexible positioning and usage of the at least one sensor is possible.

For example, in one embodiment, the apparatus comprises a pliable headgear that comprises said at least one sensor. For example, the headgear is a tight-fitting headgear. The term headgear is to be understood to include a headband, a cap, or other head-covering, in particular a tight-fitting head-covering.

In one embodiment, the apparatus comprises a pillow that comprises said at least one sensor. The term pillow is to be understood to include any support for the head of a reclining sleeping object. For example, the pillow may be integrated in a bed or a couch. For example, the pillow is a pliable pillow.

In one embodiment, the at least one sensor comprises at least one contactless sensor. For example, the at least one sensor is at least one contactless sensor. That is, the sensor is adapted to capture a bio-physiological signal of a sleeping object without electrical contact to the sleeping object, in particular, without electrical contact to the skin of the sleeping object. For example, the sensor allows to capture a bio-physiological signal of a sleeping object while there is a non-conducting space or gap between the sensor and the sleeping object. For example, the gap may contain hair. Thus, for example, the apparatus may allow to capture a brain wave signal through the hair of a sleeping object. For example, the at least one bio-physiological signal is captured through a non conductive space above the skin of the sleeping object.

Alternatively, the sensor may comprise an electrode for contacting the skin of the sleeping object.

In one embodiment, the at least one sensor is covered by a surface layer of the pliable device mentioned above. The surface layer is, for example, a lining, a pillow case, or a similar cloth, or a pliable sheet. Thus, the pliable device may provide increased comfort.

In one embodiment, the output signal is a two-dimensional graphical signal, e.g. a visual signal in analogue or digital form. For example, the output unit may comprise a display for displaying the output signal. Thus, the output signal may be visualized. Reproducing the output signal on a display of an output unit is one example of reproducing the output signal in a human-perceptible form. For example, the two-dimensional graphical signal may comprise a temporally varying color pattern and/or intensity pattern. For example, the graphical signal may be synchronized and/or correlated to at least one bio-physio logical signal, such as a brain wave signal.

In one embodiment, the output signal comprises a color signal having a temporally varying intensity and/or hue. For example, such output signal may be reproduced in a human-perceptibly form by a light source. Thus, the output unit may be a light source. For example, the output unit may be adapted to control an intensity and/or a hue of light emitted by the light source. For example, a color pattern and/or an intensity pattern of the signal may be synchronized and/or correlated to a bio-physio logical signal, such as a brain wave signal.

In one embodiment, the output signal comprises an audio signal or is an audio signal. For example, the audio signal is an analog or digital audio signal. For example, the output unit may comprise a speaker, headset, earphone, headphone or similar. Thus, a bio-physiological signal may be made audible.

In a second aspect of the invention, a method of processing a bio-physiological signal is provided, the method comprising the steps of:
- capturing at least one bio-physiological signal of a sleeping object; and
- processing the at least one bio-physiological signal using a processing unit, thereby generating an output signal based on the at least one bio-physiological signal and based on a signal pattern stored or generated in the processing unit, the output signal comprising a temporally varying output signal pattern, wherein the output signal pattern depends on a current sleep state of the sleeping object.

For example, the bio-physiological signal is a brain wave signal.

For example, the method comprises the step of reproducing the output signal in a human-perceptibly form.

For example, the output signal is correlated to at least one bio-physiological signal captured by said at least one sensor.

For example, the at least one bio-physiological signal is captured using a pliable device comprising at least one sensor, such as a pliable headgear.

For example, the at least one bio-physiological signal is captured using a pillow comprising at least one sensor.

For example, the at least one bio-physiological signal is captured using at least one contactless sensor. That is, the sensor is adapted to capture a bio-physiological signal of a sleeping object without requiring contact to the sleeping object.

For example, the output signal is a two-dimensional graphical signal.

For example, the output signal comprises a color signal having a temporally varying intensity and/or hue.

For example, the output signal is an audio signal.

For example, the method is a method of processing a bio-physiological signal using an apparatus as described above, wherein said at least one sensor captures said at least one bio-physiological signal of a sleeping object, and wherein the processing unit performs the processing step.

In a further aspect of the invention, there is provided a computer program or computer program product for performing the steps of the method as described above when executed on a computer. In particular, the computer program or computer program product may be adapted for performing the processing step and, optionally, the output step, when executed on a computer, at least one sensor for capturing at least one bio-physiological signal of a sleeping object being connected to the computer.

### BRIEF DESCRIPTION OF THE DRAWINGS

- Fig. 1: schematically shows an apparatus for processing a bio-physiological signal, having a processing unit and devices comprising sensors;
- Fig. 2: schematically shows a pillow comprising sensors;
- Fig. 3: schematically shows a wireless transmission of bio-physiological signals from a device comprising sensors to the processing unit;
- Fig. 4: schematically shows a transmission of bio-physiological signals from a device comprising sensors through a wire connection to the processing unit;
- Fig. 5: schematically shows a pliable headband comprising sensors;
- Fig. 6: schematically shows pliable headbands having integrated sensors and processing units; and
- Fig. 7: schematically shows a baby cap having sensors.

### DETAILED DESCRIPTION OF EMBODIMENTS

Various embodiments of the apparatus and method of the present invention will now be described with reference to the figures.

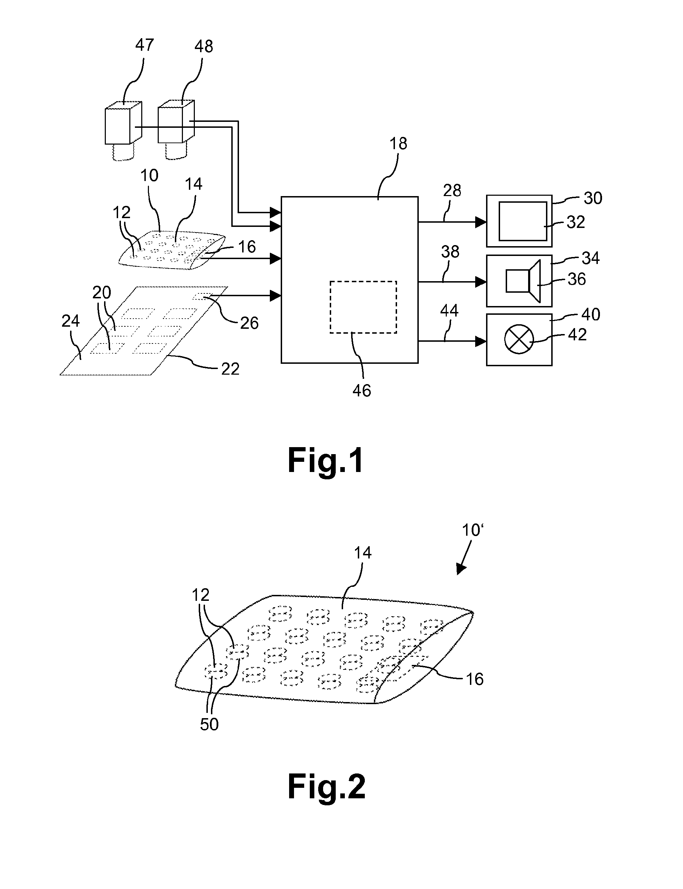

A method of processing a bio-physio logical signal will now be described in conjunction with an apparatus for processing a bio-physiological signal shown in Fig. 1. In Fig. 1, signal paths and/or signal connections are indicated by arrows.

The apparatus comprises a pliable device in the form of a pillow 10 that comprises multiple sensors 12 for capturing brain wave signals of a sleeping person, whose head rests on the pillow 10. The sensors 12 are contactless sensors, so that it is not required that the sensors 12 are in direct contract with the skin of the person. The sensors 12 are arranged in rows and columns below a cloth surface layer 14 of the pillow 10.

The pillow 10 further comprises a communication unit 16 to which the sensors 12 are connected. For example, the communication unit 16 is a wireless communication unit adapted to transmit the brain wave signals captured by the sensors 12 to a processing unit 18 of the apparatus. For example, the processing unit 18 may comprise a computer or may be a computer.

Further, electro-myogram sensors 20, for example in the form of conducting foils, are integrated in a pliable device in the form of a bed sheet 22. The electro-myogram sensors 20 are adapted to capture electro-myogram signals of a person sleeping on the bed sheet 22. For example, the electro-myogram sensors 20 are contactless sensors. For example, the sensors 20 are covered by a cloth surface layer 24 of the bed sheet 22. For example, the bed sheet 22 comprises a communication unit 26, to which the electro-myogram sensors 20 are connected. For example, the communication unit 26 is adapted to transmit electro-myogram signals to the processing unit 18 by wireless communication. Alternatively, a wire connection may be provided between the communication unit 26 and the processing unit 18. Thus, the communication unit 26 may be connected to the processing unit 28 via a wireless connection or a wired or wire connection.

The pillow 10 and the bed sheet 22 are two examples of devices comprising sensors for capturing at least one bio-physiological signal of the sleeping object. Other devices and sensors may be provided additionally or alternatively for capturing a bio-physiological signal of the sleeping object. Moreover, in a modified embodiment only brain wave signal sensors may be present, or only electro-myogram sensors 20 may be present, optionally combined with at least one sensor for capturing a different bio-physiological signal.

The processing unit 18 is adapted to process the at least one bio-physiological signal captured by the sensors 12 and/or the sensors 20 and generate an output signal as will be described below.

One or more brain wave signals may be captured by the sensors 12. For example, signal readings of more than one sensor 12 may be combined to provide a combined brain wave signal. Additionally or alternatively, one or more sensors 12 may be selected for providing at least one brain wave signal transmitted to the processing unit 18. Further, for example, the processing unit 18 may select and/or combine signal readings from different sensors 12 and/or different sensors 20 for processing. For example, sensors or sensor readings/signals may be selected depending on their respective signal amplitude or intensity.

For example, the processing unit 18 may generate an output signal 28 in the form of a two-dimensional graphical signal that is transmitted to an output unit 30 comprising a display 32. For example, the output signal 28 comprises, for at least one position or for a subset of spatial positions in the two-dimensional signal, a color signal having a temporally varying intensity and/or hue. For example, brain wave signals captured by the sensors 12 may be transformed into the output signal 28 and visualised on the display 32 using a visualisation algorithm similar to visualisation algorithms for visualising music, which are known from current music players, e.g. software music players. For example, the graphical output signal may be synchronized with a brain wave signal, so that the graphical output signal is at least partially correlated with a brain wave signal captured by the sensors 12. For example, graphical patterns may be generated in the processing unit and may be synchronised with the brain wave signal. Thus, the output signal is generated based on at least one brain wave signal captured by the sensors 12 and the signal pattern generated in the processing unit 18. Because the output signal is synchronised or correlated with a brain wave signal, a temporally varying output signal pattern depends on the brain wave pattern and, thus, on a current sleep state of the sleeping person. By generating the two-dimensional graphical signal and displaying it on the display 32, the output signal and, thus, the brain wave signal(s) captured by the sensors 12 are reproduced in a human-perceptible form. For example, during non-REM sleep, steadily evolving or substantially uniformly repeating signal patterns may be generated by the processing unit 18, whereas during REM sleep, rather irregular or uneven animated patterns may be generated, reflecting, for example, a vivid dream activity.

Additionally or alternatively to the output unit 30 having the display 32, an output unit 34 having a speaker 36 may be provided, and additionally or alternatively to generating the output signal 28, the processing unit 18 may generate an output signal 38, which may be transmitted to the output unit 34. For example, the output signal 38 is an audio signal and is reproduced by the output unit 34 in a human-perceptible form by making it audible.

One example for generating an audio output signal based on at least one brain wave signal captured by the sensors 12 and based on a signal pattern stored in the processing unit 18 is modulating a noise signal by at least one brain wave signal captured by the sensors 12. For example, pink noise, which may be generated in the processing unit 18 or which may be stored as a digital sound in the processing unit 18, may be multiplied with a brain wave signal, thereby generating the output signal. Thus, an output signal pattern, e.g. an amplitude pattern or intensity pattern of the pink noise may directly correspond to a temporally varying brain wave signal pattern. Because the brain wave pattern depends on a current sleep state of the sleeping person, the audio output signal pattern also depends on the current sleep state. In particular, the audio output signal is synchronised and correlated with a brain wave signal.

For example, during a deep sleep stage, the audio output signal may be a slowly changing waveform, similar to the sound of waves in the sea, with a deep modulation of the resulting envelope. While the person is awake, there is, for example, a faster modulation. For example, the amplitude of the output signal 38 may be controlled in amplitude and/or in dynamic range etc.

Additionally or alternatively to providing the output unit 30 and/or the output unit 34, an output unit 40 comprising at least one light source 42 such as a lamp may be provided. Further, additionally or alternatively to providing the output signal 28 and/or the output signal 38, an output signal 44 may be generated by the processing unit 18 as follows and transmitted to the output unit 40. For example, the output signal 44 may be a color signal and/or intensity signal for controlling the hue and/or intensity of light emitted by the light source(s) 42 of the output unit 40. For example, the output signal 44 may comprise RGB values. For example, the light source 42 may comprise one or more LEDs. The output unit 40 is, for example, a device for creating ambient light.

For example, a color and/or intensity signal component of the output signal 44 will be generated in a manner similar to generating the output signal 28 as described above. For example, the color and/or intensity may correspond to an instantaneous average color or intensity of the output signal 28, e.g. a mean color or intensity of a two-dimensional image or video frame.

In another example, the processing unit 18 may perform a frequency analysis of brain wave signals captured by sensors 12, and, depending on the occurrence of frequencies that are characteristic for a specific sleep stage, the processing unit 18 may select a color and/or intensity output signal pattern assigned to that sleep state. For example, a frequency of an intensity variation of the output signal 44 may be selected depending on a detected sleep state. For example, a deep sleep stage may be indicated by green or blue light of a slowly varying intensity, whereas, when an REM sleep stage is detected, the output signal 44 may comprise a color-changing pattern.

The frequency analysis or sleep stage detection may be performed by a sleep stage detection unit 46 of the processing unit 18, such as a sleep stage detection algorithm in the processing unit 18. Detecting the sleep stage may be based on characteristic features of brain wave signals detected by the sensors 12 and/or characteristic features of electro-myogram signals detected by the sensors 20 and/or characteristic features of other suitable bio-physiological signals captured by other sensors. As described above, the output signal 44 comprising a temporally varying output signal pattern is selected depending on a current sleep state of the sleeping person. Thus, the output signal 44 is generated based on at least one bio-physiological signal and based on a signal pattern stored or generated in the process unit 18.

Other examples of generating a two-dimensional graphical output signal 28 or an audio output signal 38 based on a detected sleep stage are described in the following. For example, depending on a detected sleep state, the processing unit 18 may select a two-dimensional graphical output signal 28, which is stored or generated in the processing unit 18. For example, pre-defined patterns or pattern generating algorithms may be selected by the processing unit 18 dependent on the detected sleep stage. Thus, a temporally varying output signal pattern of the output signal 28 depends on a current sleep state of the sleeping person. However, the output signal is not necessarily synchronised or correlated with a bio-physiological signal. In a similar manner, the processing unit 18 may select an audio signal pattern stored or generated in the processing unit 18 depending on a detected sleep stage. Again, a temporally varying output signal pattern of the audio output signal 38 depends on the current sleep state of the sleeping person, whereas, however, the output signal 38 is not necessarily synchronized or correlated with a bio-physio logical signal.

The output signals 28, 38, 44 may be transmitted via a wireless connection or a wire connection to the respective output unit. Further, for example, an output unit, such as the output unit 30, 34 or 40, may be integrated into the processing unit 18.

In the embodiment shown in Fig. 1, a contactless respiration and/or heart rate sensor 47 is provided and is connected by a wire connection or a wireless connection to the processing unit 18. For example, due to the respiratory sinus arrhythmia, the heart rate may be determined from the respiratory signal.

For example, the respiration and/or heart rate sensor 47 is a photoplethysmographic imager (PPGI) including an infrared light source and, for example, a camera with an IR filter. Thus, the respiration rate may be captured even if the person lays with his mouth on a pillow. For example, the sensor 47 determines a bio-physio logical signal in the form of the heart rate and/or a bio-physiological signal in the form of the respiratory rate for processing by the processing unit 18. The heart rate and the respiratory rate may depend in a characteristic manner on a sleep state. For example, when entering REM sleep, respiration and heart rate increases substantially. Thus, the processing unit 18 may, for example, be adapted to process the at least one bio-physiological signal captured by the sensor 47 and generate an output signal as described above, similar to processing the signals from the sensors 12 and/or 20. For example, detecting the sleep stage may be based, in addition to or alternatively to the signals mentioned above, on characteristic features of the heart rate signal and/or characteristic features of the respiratory rate signal captured by the sensor 47. For example, the heart rate and/or the respiratory rate may be used as input or as additional input to the sleep stage detection unit 46. As described above, an output signal 44 comprising a temporally varying output signal pattern may be selected depending on a current sleep state of the sleeping person. In a modified embodiment, the sensor 47 may be present instead of the sensors 12 and/or 20, optionally combined with at least one sensor for capturing a different bio-physiological signal.

The respiration and/or heart rate sensor 47 may, for example, alternatively or additionally include a micro wave Doppler radar sensor adapted to provide a heart rate signal and/or a respiration rate signal, for example, by non-contact, through-clothing measurement of chest wall motion of the sleeping person.

In the embodiment shown in Fig. 1, optionally, an image acquisition device in the form of a camera 48 is provided and is connected by a wire connection or wireless connection to the processing unit 18. For example, the camera 48 is arranged above the pillow 10. The processing unit 18 may be adapted to determine, from an image signal of the image acquisition device, whether there is a head on the pillow 10, and/or to determine an orientation and/or position of the head. For example, the processing unit 18 may select sensors 12, the sensor readings of which are to be processed, based on a detection of an orientation and/or position of a head on the pillow 10. In case the sensor 47 includes a camera, this camera may as well form the image acqusition device and may be used instead of the camera 48.

Fig. 2 shows another example of a pillow 10' which, for example, may be provided instead of the pillow 10 in the embodiment of Fig. 1. The pillow 10' is similar to the pillow 10 described above and comprises, for example, the same sensors 12, cloth surface layer 14 and communication unit 16 arranged and connected as described above. However, the pillow 10' comprises pressure sensors 50 connected to the communication unit 16. Pressure signals of the pressure sensors 50 may be transmitted to the processing unit 18. The processing unit 18 is, for example, adapted to determine whether there is a head on the pillow 10' based on the pressure signals. Further, for example, the processing unit 18 may be adapted to determine an orientation and/or position of a head on the pillow 10' based on the pressure signals. Thus, additionally or alternatively to an image signal of the camera 48, pressure signals of the pressure sensors 50 may be used to determine an orientation and/or a position of a head on the pillow 10'. Thus, sensors 12, the captured signals of which are to be processed, may be selected based on the determined orientation and/or position of a head.

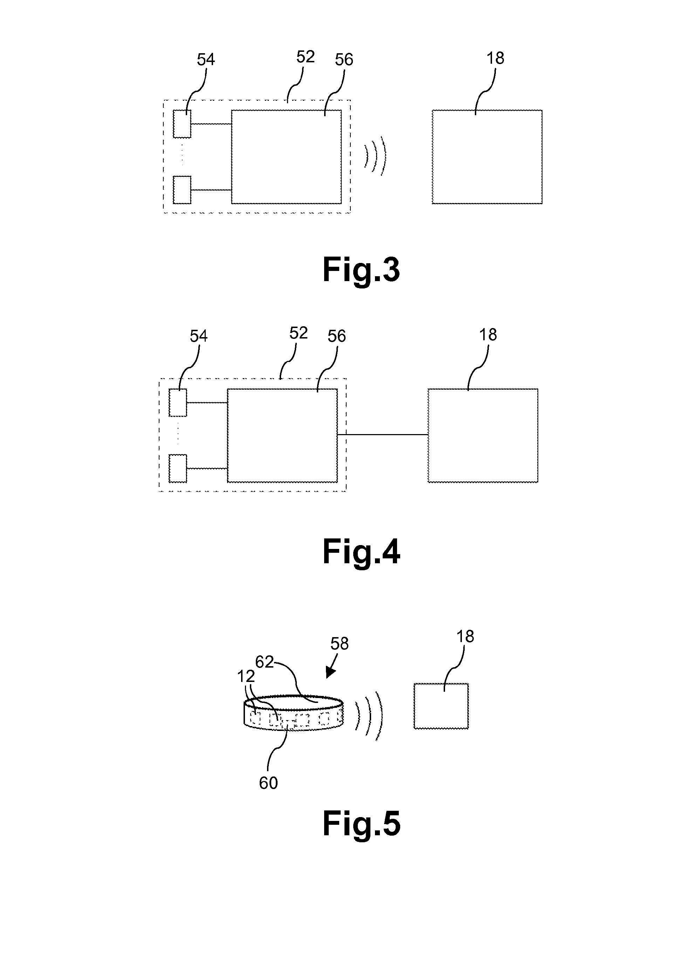

Figs. 3 and 4 schematically show a device 52 comprising at least one sensor 54 for capturing at least one bio-physiological signal of a sleeping object, and a connection of said device 52 to the processing unit 18. The sensors 54 are connected to a communication unit 56 for transmitting the bio-physio logical signals to the processing unit 18. In Fig. 3, the connection is a wireless connection, and in Fig. 4, the connection is a wire connection. More than one device 52 may be connected to the processing unit 18. For example, the device 52 may be the pillow 10 and the sensors 54 may be the sensors 12, and the communication unit 56 may be the communication unit 16. Further, for example, the device 52 may be the bed sheet 22, the sensors 54 may be the sensors 20, and the communication unit 56 may be the communication unit 26. The processing unit 18 may also be included in the device 52. For example, the processing unit 18 may be included in the pillow 10.

Fig. 5 shows an example of a pliable device 52 in the form of a head band 58 comprising sensors 12 for capturing at least one brain wave signal of a sleeping person wearing the headband 58. Further, the headband 58 comprises at least one electro-oculogram sensor 60 for capturing an electro-oculogram signal of the person. For example, the person places the headband 58 such that the EOG sensor 60 is positioned above an eye.

For example, the sensors 12 and the at least one EOG sensor 60 are connected to a communication unit 56 integrated in the head band 58 for transmitting the signals captured by the respective sensors 12, 60 to the processing unit 18 similar to the example of Fig. 3. The headband 58 is one example of pliable headgear containing at least one sensor for capturing at least one bio-physiological signal of a sleeping object. For example, the sensors 12 and 60 are contactless sensors. For example, the sensors 12 and 60 are covered by a cloth surface layer 62 of the pliable headband 58.

The headband 58 may be provided instead of or additionally to the pillow 10 and/or the bed sheet 22 of the embodiment of Fig. 1. For example, alternatively to or additionally to processing the signals of the sensors 12 of the pillow 10, the signals of the sensors 12 of the headband 58 and/or the signals of the at least one EOG sensor 60 may be processed by the processing unit 18, thereby generating an output signal as described above. Providing a headband 58 has the advantage that the capturing of the signals is less dependent on the sleeping object's position on a pillow. When the output signal 28, 38 and/or 44, for example, is generated based on an EOG signal from an EOG sensor 60 and on a signal pattern stored or generated in the processing unit 18, the rapid eye movements of the sleeping person may be made visible and/or audible. For example, a varying output signal pattern may reflect the varying eye movements during the REM sleep stage. By generating an output signal based on a brain wave signal of the sensor(s) 12 and/or the electro-oculogram signal of the sensor(s) 60, in some respects, dreams of the sleeping person may be visualised and/or made audible.

In one example, the processing unit 18 may be integrated in the device 52, such as the headband 58. For example, the device 52, such as the headband 58, may comprise the processing unit 18 as well as an output unit, such as the output unit 34. For example, the headband 58 may comprise speakers 36 in the form of earphones or headphones in order to make an audio output signal audible. For example, there is a wire connection between the sensors 12, 60 and the processing unit 18. Thus, the apparatus for processing a bio-physiological signal may be a headband 58 comprising the processing unit 18 and, optionally, an output unit. For example, the headband 58 may comprise batteries for powering the apparatus. The headband 58 is one example for a portable apparatus for processing a bio-physiological signal.

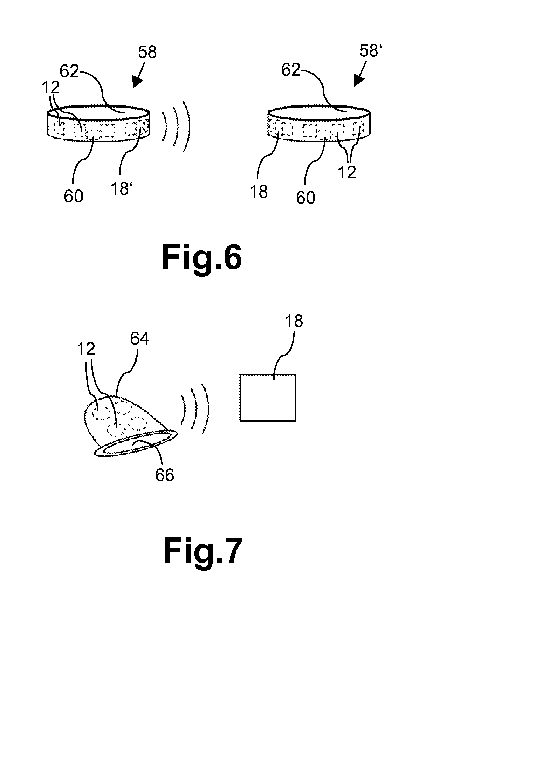

In Fig. 6, a system for processing bio-physiological signals is shown comprising two headbands 58, 58', each having the sensors 12 and 60 as described for the embodiment of Fig. 5. In the embodiment of Fig. 6, the communication unit 18 for processing the signals of the sensors of the headband 58 is included in the headband 58'. Correspondingly, a processing unit 18' for processing the signals of the sensors of the headband 58' is included in the headband 58. The sensor signals are transmitted via a wireless connection between respective communication units 56 of the respective headbands 58, 58'. Further, for example, output units 34 and/or output units 40 may be included in the respective headbands 58, 58'. Thus, brain wave signals and/or electro-oculogram signals of a sleeping person wearing a headband 58 may be wirelessly transmitted to a headband 58' of a partner in order to visualise them or make them audible.

In an alternative example, the processing unit 18, 18' for processing the sensor signals of the respective headband 58, 58' may be included in said headband 58, 58', and the output signal generated by the respective processing unit may be wirelessly transmitted to an output unit 34 or 40 included in the other headband 58', 58.

Thus, in the examples of Fig. 6, a system for processing bio-physiological signals is provided, said system comprising two processing units for processing at least one bio-physiological signal captured by a respective at least one sensor associated with said processing unit. In this sense, the system combines two apparatuses for processing a bio-physiological signal as described above in conjunction with Fig. 1.

Fig. 7 shows an embodiment where the device 52 is a pliable baby cap 64 comprising sensors 12 for capturing at least one brain wave signal of a sleeping baby wearing the baby cap. For example, the sensors 12 are covered by a cloth surface layer 66 of the baby cap 64. Similar to the example of Fig. 3, a communication unit 56 may be included in the baby cap 12 for communication with the processing unit 18. For example, the processing unit 18 and at least one output unit, such as an output unit 40, may be included in a baby crib. This is an example of the embodiment of Fig, 1, wherein the baby cap 64 may be provided additionally or alternatively to the pillow 10. For example, the visualisation of an output signal of the processing unit 18 may be done by using lighting elements of the output unit 40 embedded in the crib. Thus, for example, if the baby was sleeping well, the crib could light up a green glow light pattern, and if the baby is restless, the crib could light up in a different pattern and/or a differently colored pattern.

As described above, for example, the invention may allow to visualise a sleep state, make it audible or, in general, produce a human-perceptible output signal related to a sleep state or dream of the sleeping object. For example, the invention may enable people to see that their baby, child or loved-one is sleeping well. Further, for example, the invention may allow making their sleep and/or dreams tangible in some way. For example, an apparatus for consumer use that enables a person to monitor another person's sleep in an interesting and/or entertaining manner is provided.

## Claims

1. Apparatus for processing a bio-physio logical signal, comprising:
- at least one sensor (12; 20; 60) for capturing at least one bio-physiological signal of a sleeping object; and
- a processing unit (18) for processing the at least one bio-physio logical signal and thereby generating an output signal (28; 38; 44) based on the at least one bio-physiological signal and based on a signal pattern stored or generated in the processing unit (18), the output signal (28; 38; 44) comprising a temporally varying output signal pattern, wherein the output signal pattern depends on a current sleep state of the sleeping object.

2. Apparatus as claimed in claim 1, wherein the bio-physiological signal is a brain wave signal.

3. Apparatus as claimed in claim 1 or 2, further comprising an output unit (30; 34; 40) for reproducing the output signal in a human-perceptible form.

4. Apparatus as claimed in any one of claims 1 to 3, wherein the output signal (28; 38; 44) is correlated to at least one bio-physiological signal captured by said at least one sensor (12; 20; 60).

5. Apparatus as claimed in any one of claims 1 to 4, further comprising a pliable device (10; 22; 58; 64) that comprises said at least one sensor (12; 20; 60).

6. Apparatus as claimed in any one of claims 1 to 5, further comprising a pliable headgear (58; 64) that comprises said at least one sensor (12; 60).

7. Apparatus as claimed in any one of claims 1 to 6, further comprising a pillow (10) that comprises said at least one sensor (12).

8. Apparatus as claimed in any one of claims 1 to 7, wherein the at least one sensor (12; 20; 60) comprises at least one contactless sensor (12; 20; 60).

9. Apparatus as claimed in any one of claims 1 to 8, wherein the output signal (28) is a two-dimensional graphical signal.

10. Apparatus as claimed in any one of claims 1 to 9, wherein the output signal (28; 44) comprises a color signal having a temporally varying intensity and/or hue.

11. Apparatus as claimed in any one of claims 1 to 10, wherein the output signal (38) is an audio signal.

12. Method of processing a bio-physiological signal, comprising the steps of:
- capturing at least one bio-physiological signal of a sleeping object; and
- processing the at least one bio-physiological signal using a processing unit (18), thereby generating an output signal (28; 38; 44) based on the at least one bio-physiological signal and based on a signal pattern stored or generated in the processing unit (18), the output signal (28; 38; 44) comprising a temporally varying output signal pattern, wherein the output signal pattern depends on a current sleep state of the sleeping object.

13. Computer program or Computer program product for performing the method as claimed in 12 when executed on a computer.

14. Data Carrier including a Computer program for performing the steps of the method as claimed in claim 12.

15. Computer for executing a computer program as claimed in claim 13.
